# EUROPEAN PATENT SPECIFICATION

(11) **EP 1 265 561 B1**
(45) Date of publication and mention of the grant of the patent: **08.07.2009**
(21) Application number: 01916659.4
(22) Date of filing: 14.03.2001
(51) Int. Cl.: A61F 2/44

(54) **SYNTHETIC THREADED VERTEBRAL IMPLANT**
WIRBELSÄULENIMPLANTAT MIT GEWINDE UND AUS SYNTHETISCHEM MATERIAL BESTEHEND
IMPLANT VERTEBRAL SYNTHETIQUE FILETE

(30) Priority: 14.03.2000 DE 20004692 U
(43) Date of publication of application: 18.12.2002
(73) Proprietor: Warsaw Orthopedic, Inc., Warsaw, IN 46581 (US)
(72) Inventor: FRÜH, Hans-Joachim, 94469 Deggendorf (DE); EBNER, Harald, 94469 Deggendorf (DE); ESTES, Bradley, T., Durham, NC 27705 (US)
(74) Representative: Viering, Jentschura & Partner
(86) International application number: PCT/US2001/008193
(87) International publication number: WO 2001/068006

(56) References cited:
- WO-A-96/40020
- WO-A-97/37619
- DE-U- 29 612 271
- US-A- 5 766 253
- US-A- 5 895 427

## Description

### CROSS-REFERENCE TO RELATED APPLICATION

The present application claims the benefit of German Utility Model Application No. 200 04 692.6 filed on March 14, 2000.

### FIELD OF THE INVENTION

The invention relates to a vertebral implant according to the preamble of claim 1. Further, the invention relates to a method according to the preamble of claim 21.

In general this invention relates to a synthetic vertebral implant and methods of manufacturing and using the implant. More specifically but not exclusively, this invention is directed to a synthetic, threaded vertebral implant suitable for restoring and or maintaining desired disc space height.

### BACKGROUND OF THE INVENTION

A vertebral implant and a method of the initially - mentioned type are known, e.g., from WO 97/37619 A.

For degenerated, diseased or otherwise damaged spinal columns and vertebrae, it is known to treat these defects by removal of all or a portion of the vertebral disk and inserting an implant such as a spinal spacer into the disc space to restore normal disk height and spine orientation, and repair the spinal defects. When desired, osteogenic material also can be implanted into the intervertebral space to promote arthrodesis, or spinal fusion between the two vertebrae adjacent to the intervertebral space. Selected spacers are formed to provide a cavity for receipt of the osteogenic material.

The spinal column can exert tremendous force on the individual vertebrae, and consequently also on any implant implanted in between the vertebrae. Spinal implants typically are formed of a metal such as titanium or surgical steel. While the selection of the implant configuration and composition can depend upon a variety of considerations, for arthrodesis it is often desirable to select a material that does not stress shield the bone ingrowth. Titanium and surgical steel provide the requisite strength to maintain correct disk space height and orientation; however, these materials have been shown to stress shield the bone. Bone and bone derived material can provide an acceptable material having the similar strength and compressibility as living bone tissue. However, suitable donor bone is scarce. Further, extensive screening and sterilization must be strictly observed to minimize the risk of transmission of infections, either real or perceived, from the donor to the recipient.

The following patents are representative of the current state of the art for the relevant technology.

In United States patent 5,669,909 issued to Zdeblick et al. disclosed an interbody fusion device for threaded insertion into the intervertebral space. The device has a generally elongate, conical body defining a series of interrupted external threads The elongate body has two truncated or flattened sidewalls diametrically opposed to each other. The truncated sidewalls are touted to facilitate insertion of the implant into the intervertebral space. The body encloses a cavity for receipt of bony material. The device is inserted into the disk space so the opposing truncated sidewalls bear against the endplate of adjacent vertebrae. Once inserted, the device is turned 90° to engage the interrupted threads with the bone tissue of the endplates.

Brosnahan in U.S. Patent 5,766,253 discloses a solid spinal fusion device having a threaded exterior. The device includes two indentions on its outer surface for bone attachment material. This reference mentions that the fusion device can be formed of a biocompatable osteoconductive material such as bioactive hydroxyapatite-polymer composites, preferably a hydroxyapatite reinforced polyethylene composite.

Bagby in U.S. Patent No. 6,010,502 discloses a metallic cylindrical base body having a helically configured spline or thread configured on its outer surface. The body can have a hollow interior. Large and small circular fenestrations extend from the surface into the hollow interior. The metallic body can be fitted with a plastic cap to protect the spinal cord from abrading against the end of the metallic body.

There remains a continuing need for advancements in the relevant field, including treatment of damaged or diseased spinal columns, improved implants, selection of suitable materials from which the implants are formed and methods of enhancing the bone fusion between adjacent vertebrae. The present invention is such an advancement and provides a wide variety of benefits and advantages.

### DISCLOSURE OF INVENTION

The invention provides a vertebral implant according to claim 1. Further, the invention provides a method according to claim 21. Further embodiments of the invention are described in the dependent claims.

The present invention relates to spinal implants, the manufacture and use thereof to treat degenerated, diseased or otherwise damaged spinal columns. Various aspects of the invention are novel, nonobvious, and provide various advantages. While the actual nature of the invention covered herein can only be determined with reference to the claims appended hereto, certain forms and features, which are characteristic of the preferred embodiments disclosed herein, are described briefly as follows.

The invention provides a vertebral implant capable of being threaded into an intervertebral space and which is stable with respect to the expected biomechanical forces. In one embodiment, the implant or intervertebral spacer can be integrated into the bony tissue. In alternative embodiments the implant or spacer is biodegradable. The implants according to this invention can be manufactured at low cost.

The vertebral implant to be screwed into an intervertebral space according to one aspect of this invention and comprises a hollow cylindrical base body arranged to receive bone material and provided with an external thread so that the base body can be threadedly implanted by engaging the two vertebrae defining the intervertebral space or disc space. The vertebral implant further comprises two holes preferably located diametrically opposing each other and extending across several thread ribs in the longitudinal direction of the base body so as to interrupt the course of the thread ribs. The holes are preferably elongated. Although it will be understood, that the holes can be provided in a wide variety of configurations. The holes allow a bone bridge to form between the two vertebrae and through the implanted base body. The two circumferential wall portions of the base body disposed between the holes are each provided with at least one longitudinal through-slit extending in the longitudinal direction of the base body and interrupting, at least partially, the course of the thread ribs. Each longitudinal slit is narrower and shorter than the elongated holes, and is designed to enable tissue lateral of the implanted base body to grow sideways into the interior of the base body. The vertebral implant is preferably made of a synthetic material, for example, a polymeric material, a composite, a ceramic or a reinforced material.

To insert the vertebral implant into an intervertebral space according one embodiment of this invention, the implant is screwed or threaded into the intervertebral space. Thus, unlike an implant that has to be pushed, driven or impacted, the risk of the implant being suddenly displaced to an unintended position is minimized. Rather the implant can be gradually rotated and screwed axially forward into the intervertebral space and, thus, positioned accurately in the intervertebral space without any hazard. To this end, the thread of the vertebral implant preferably has a small lead angle, advantageously less than about 10°, more preferably between about 2° and about 8°. Owing to its simple integral design, the implant has a smooth, tapering--albeit threaded profile, i.e. lacking any projections, so there is hardly any risk of hurting surrounding tissue when the implant is being screwed into the intervertebral space. This integral, compact and still sufficiently stable construction of the implant further allows it to be made of a synthetic material, for example, a reinforced material, a polymeric materiel, a composite or a ceramic. These materials are preferred for a variety of advantageous benefits including low cost, long durability, strength and good biocompatability.

In its implanted state, the implant is positioned in the intervertebral space such that the two elongated holes face the respective vertebrae so that a bone bridge can build up between these vertebrae through the elongated holes and the vertebral implant, i.e., spinal fusion of the adjacent vertebrae.

Lateral portions of the circumferential wall are reinforced by thread ribs. The vertebral implant constitutes a sufficiently stable support to receive the biomechanical forces occurring between the two vertebrae until the complete bone bridge has formed. As the elongated holes and slits are formed directly in (or through) the thread, the circumference for the base body is provided with thread ribs over a maximum surface of its outer periphery, thus strengthening the circumferential walls of the implant.

This design increases the stability of the implant. At the same time, this design enables the vertebral implant to be fixed in a reliable manner because the growing bone tissue intimately engages the thread ribs, which are interrupted completely by the elongated holes and at least partially by the longitudinal slits. Moreover, the number of manufacturing steps is also reduced. The slits arranged in the two circumferential wall portions between the elongated holes form a sufficiently large passage for vascular tissue lateral of the implanted implant to grow into the implant, thus improving the nutritional supply of the bone material accommodated in the implant. At the same time, however, the slits are sufficiently small not to jeopardize the stability of the implant. In addition, this enabled lateral nutritional supply stimulates the bone tissue in the implant to also grow from inside into the lateral slits thus further improving the fit of the implant.

The reinforced implant can include a wide variety of reinforcing materials included fibers, platelets, and/or particulate elements. The fiber-reinforced implant material may be embodied by glass fibers, ceramic fibers or carbon fibers. Preferably, the implant material comprises long carbon fibers, in particular endless carbon fibers, allowing a predetermined high strength to be achieved at low manufacturing cost.

The implant can also be formed of a polymeric material, for example, polyanhydrides; polyamides, poly(amino acids), polycaprolactones, polylactate, poly(lactide-co-glycolide); polyorthoesters; acrylics; polycarbonates; polyesters; polyethers, poly(ether ketone); poly(ether, ether ketone) (PEEK); poly(aryl ether ketones) (PAEK; poly(ether ether ketone ether ketone) (PEEKEK); poly(ethylene terephthalate) (PET), poly(acrylate) poly(methyl (meth)acrylate), polyolefins, polysulfones, polyurethane; poly(vinyl chloride), epoxy resins, carbon reinforced composites, glass reinforced composite, ceramic reinforced composites, and mixtures thereof. Alternatively the implant can be formed of a ceramic, for example, a material selected from the group of: hydroxylapatite; alumina, zirconia and mixtures thereof.

Each longitudinal through-slit preferably extends across at least one rib of the thread. The bone material can grow sideways through the longitudinal through-slit and through a complete gap of the course of the thread rib of the implant. This inhibits axial rotation of the implant. The inhibition of axial rotation of the implant is even enhanced with respect to a situation where bone material can primarily grow sideways around the outside parameter of the implant between two adjacent thread ribs.

The base body may be circular or slightly conical or may have at least one conical end portion. Preferably, the overall shape of the base body is cylindrical; this can allow it to be manufactured even more easily and at lower costs. A separating force (or distraction) can be exerted on the vertebrae; this can be achieved by distraction during surgery using distractors. Additionally the implant itself can provide distraction by selecting a base body having a diameter greater than the existing disc space height between the adjacent vertebrae. The selected implant can be threaded into the disc space. The threads of the implant engage in the opposing surfaces of the vertebrae, and pulling the implant into the disc space and consequently distracting the disc space as the implant becomes fully seated or positioned at a desired position within the disc space.

In other embodiments the base body can define a lordotic profile. In this configuration the circumferential wall portions are shaped to provide a spacer that conforms to the desired lordosis or natural curvature of the spine. In one form the circumferencetial wall portions are formed as conical wall portions while still retaining an exterior thread.

The longitudinal openings or slits can be arranged at any place in the circumferential wall portions, it is preferred that the longitudinal openings or slits oppose each other diametrically. It will be understood that one, two, three or more pairs of longitudinal openings can be provided in the circumferential wall portions of the implant. This arrangement allows the vascular tissue lateral of the implanted implant to communicate with the osteogenic material deposited in the implant so that the nutritional supply of the bone material is greater and more homogeneous. The blood supply, and thus, the supply of nutrition to the bone tissue growing through the implant are further improved enabling the implant to be integrally incorporated in the growing bone tissue.

It is further preferred that at least one longitudinal slit is disposed in the circumferential wall at an angular distance of about 90° from the respective elongated hole, as seen in the circumferential direction of the base body. This design allows the vascular tissue a more direct path into the implant. An additional advantage resides in that the bone tissue can grow orthogonally through the implant resulting in a particularly stable crosswise anchoring of the implant or pair of implants.

A preferred embodiment provides two longitudinal slits in each circumferential wall portion between the elongated holes of the base body. The two longitudinal slits are disposed at a distance from each other in the longitudinal direction of the base body. Owing to this design, a circumferential web remains between the two longitudinal slits in the axial direction of the implant and ensures sufficient stability of the implant with respect to the compressive forces to be received. The circumferential web between the two longitudinal slits is preferably wide enough to carry at least one uninterrupted thread rib, preferably two or more interrupted thread ribs, thereon. It is further preferred for the slits to be arranged symmetric with respect to the longitudinal center of the base body. This can facilitate the osteogenic material or bone material within the implant to contact vascular tissue from the lateral side of the implant as far as possible over the entire length of the implant without jeopardizing the inherent stability of the implant.

The thread may be embodied by any type of thread, such as a sharp, triangular, or rounded-over thread. It is preferred, however, that the external thread be formed as a trapezoidal thread. The natural thickness of the ribs of a trapezoidal thread inhibits the implant from sinking or subsiding into the vertebrae, particularly into degenerative vertebrae. Further, the wide thread ribs also increase the reinforcement of the circumferential wall. According in a preferred embodiment of the invention the average thickness of the thread ribs is in the range of between about 1/25 to 1/15 of the overall length of the implant, more preferably about 1/20 of the overall length of the implant.

In order to facilitate the screwing of the implant into a prepared threaded bore between the vertebrae and also to facilitate the threading operation on the implant during manufacture thereof, the two axial ends of the base body are preferably provided with beveled edges. The beveled edges may be tapered or round chamfers, for example. According to a preferred embodiment, the axial front end portion of the base body is provided with an insertion end tapering axially from the larger first, outer diameter of the implant to a smaller second, outer diameter proximate the front end. The insertion end can be arranged to be set onto the vertebrae when the implant is threaded into the intervertebral space. This particularly applies to cases where the vertebrae are to be spread apart by means of the implant to a larger extent, i.e., using the implant itself to distract the adjacent vertebrae. In an alternative form, the insertion end does not include an external thread. To this end, initially the implant can be easily driven or impacted into the intervertebral space to initially spread the vertebrae apart and to enter the intervertebral space until the thread on the circumferential wall portion engages the opposing end plates or surfaces of the adjacent vertebrae. In still other alternative embodiments, the insertion end includes a threaded portion. In this embodiment, the implant can be threadedly implanted into the disc space without the necessity of impaction. Regardless, after initial placement the implant can be positioned accurately in the intervertebral space by screwing the implant in the axial direction, with reduced risk of the implant damaging or penetrating adjacent tissue or structures, for example, the spinal cord. The implant can include a set-head, which is preferably at least 1/10 of the base body in the axial direction thereof.

While the implant may be gripped manually or with a tong-type tool and threaded into the intervertebral space in any manner, the axial rear end of the base body advantageously comprises a receiving means or tool engaging portion for receiving a manipulation tool in order to exert a torque on the base body. This receiving means enables a more accurate implantation process, and minimizes damage to the implant during surgery, which finally better ensures a durable functionality of the implant in the implanted state thereof.

In one embodiment the receiving means can be embodied by two or more holes, for example, arranged in the front wall of the base body. The holes are provided for mating engagement with matching pins of the manipulation tool. A torsional moment torque can be exerted on the implant by rotating the engaged manipulation tool. In other embodiments, the receiving means preferably comprises two grooves opposite each other with respect to the cavity of the base body, and a mating blade or a matching counterpart on the manipulation tool can engage these grooves from outside. The manipulation tool can be prevented from slipping inadvertently off and away from the end of the implant by providing an engagement groove or other engagement means with an undercut portion or a dove tail, for example, that can be detachable locked or engaged with a matching counterpart overcut portion of the manipulation tool.

Preferably, the receiving means also comprises a threaded central bore into which a corresponding threaded counterpart of the manipulation tool. for example, a threaded pin can be screwed in such a manner that the manipulation tool is firmly engaged to the implant. This provides the advantage that the implant is coupled integrally to the manipulation tool and can be manipulated accurately together with the manipulation tool by the surgeon.

The above-described implants can be prepared of a wide variety of materials including synthetic organic materials, composites, and ceramics. Preferably the implants are formed of a synthetic, non-metallic material. The implants of the present invention can be either essentially permanent implants, which do not readily biodegrade. These implants can remain in the intervertebral space and often are incorporated into the bony tissue. Alternatively, the implant can biodegrade or erode over time and are substantially replaced by bone tissue.

Examples of nondegradable polymeric or oligomeric materials include the, polyacrylates, polyethers, polyketones, polyurethanes, epoxides and copolymers, alloys and blends thereof. Use of the term co-polymers is intended to include within the scope of the invention polymers formed of two or more unique monomeric repeating units. Such co-polymers can include random copolymers, graft copolymers, block copolymers, radial block, diblock, triblock copolymers, alternating co-polymers, and periodic co-polymers. Specific examples of nondegradable polymeric materials include: poly(vinyl chloride) (PVC); polyacrylates, poly(methyl (meth)acrylate); acrylics; polyamides; polycarbonates; polyesters; polyethylene terephthalate; polysulfones; polyolefins, i.e. polyethylene, polypropylene, and UHMWPE (ultra high molecular weight polyethylene); polyurethane; polyethers, i.e., epoxides; poly(ether ketones) (PEK), poly(ether, ether ketones) (PEEK), poly(aryl ether ketones) (PAEK), and poly(ether ether ketone ether ketone) (PEEKEK). A wide variety of suitable poly(ether-co-ketone) containing materials are commercially available.

Alternatively, implants of this invention can be made of a material that either biodegrades or is bioabsorbed. Typically, biodegradable material is a polymeric material or oligomeric material and often the monomers are joined via an amide linkage such as is observed in poly(amino acids). When the implant is formed of material that biodegrades, it is desirable to provide a biodegradable material that degrades at a rate comparable to the bony ingrowth characteristic of bone fusion often referred to as creeping substitution. It is still more preferred to select the biodegradable material to remain *in situ* and capable of providing sufficient biomechanical support for the spine even after a bone bridge has grown and formed through the through-holes of the implant. The biodegradation rate of the implant can be varied by selecting an appropriate synthetic material. The degradation rate of the selected material can be further modified; for example, the degradation rate can be decreased by increasing the amount of crosslinking between the polymer chains and/or the increasing the degree of polymerization. Further, it is not intended to limit the preferred materials to substances that are partly or totally reabsorbed within the body. Rather substances that can be broken down degraded and eventually flushed from the body are also intended to come within the scope of this invention.

Examples of biodegradable polymers for use with this invention include poly(amino acids), polyanhydrides, polycaprolactones, polyorthoesters, polylactic acid, poly(lactide-co-glycolide), i.e., copolymers of lactic acid and glycolic acid, including either D, L and D/L isomers of these components. One example of a preferred biodegradable polymer for use with this invention is a copolymer of 70:30 poly(L, DL) lactate commercially available from Boehringer Ingelheim.

A particularly advantageous benefit provided by this invention is the ease of manufacturing suitable synthetic implants. Implants formed of polymeric, oligomeric and composite material can be manufactured using known fabricating techniques, including various extrusion, injection molding and blow molding processes. In addition, selected polymeric materials are provided by suppliers in a form that can readily formed, and/or molded, usually at an elevated temperature. A copolymer of D/L lactate is one specific example. This material can be obtained in a wide variety of forms including pellets or granules, sheets, ingots. The material can be molded at a temperature of about 55°C or greater to provide a desired shaped and sized implant. The material can be repeatedly heated and contoured without any significant change in its material or chemical properties. In addition, material is readily cut using a cautery to readily conform the implant to the bone. The lower cautery temperature even permits cutting the material during the operation.

Specific examples of ceramic materials for use with this invention include glass, calcium phosphate, hydroxyapatite, alumina, zirconia, and mixtures of these materials.

Composites are also useful with this invention. Composites can combine two or more of the desired materials to form an implant body for implantation. Examples of composites include combinations of ceramics, glass and/or polymeric materials. Preferred composites include a reinforcing material. The reinforcing material can include platelets, particulates or fibers.

The following provides an advantageous filament winding method for manufacturing an implant for use in the present invention from a fiber reinforced material, such as a glass or carbon fiber-reinforced material. First, the fibers are impregnated with a liquid synthetic material. A particularly preferred material is an epoxy resin. The impregnated fibers are wound about a winding spindle. The fibers are preferably carbon fibers. Preferably the fibers are wound on the winding spindle in the form of a bundle using a filament winding process. Thereafter the synthetic material is cured; this is preferably carried out by a controlled temperature treatment. A simple, hollow, rod-shape implant can be prepared by winding the fibers around a winding spindle having the simple rod-shape. More sophisticated shaped implants can be manufactured by a more sophisticated-shaped spindle. For example an implant having elongated through-holes can be prepared by using a spindle having corresponding protuberances to define the through-holes. The final dimensions of the implant can be defined by the dimensions of the winding spindle so that little if any machining of the inner surfaces of the walls defining the cavities is required.

In a subsequent step, the outer periphery of the base body is machined so that the implant body has a rectangular cross-section.

In a machining process, which preferably includes a milling/cylindrical grinding steps, the body is provided with a circular cross-section; longitudinal slits are formed in the circumferential wall between the two elongated through-holes; and an external thread is formed about the exterior of the circumferential wall. If desired, a smoother and often stronger surface can be obtained by corundum blasting the implant body in an additional processing step.

Optionally, the tool engaging end of the implant can provided with a transverse groove and a bore, which may or may not be threaded. The groove and bore can be machined using known techniques for drilling and/or milling processes.

In order to avoid any damage to the inner surfaces of the implant body during the drilling and/or milling process steps, i.e. in order to prevent undesirable cracks in the material, a wood core, in particular beech wood core, is inserted in the implant body in place of the winding spindle, during the processes. The wood core can be wetted to swell to the inside geometry of the implant.

This method provides a highly stable vertebral implant from fiber-reinforced material at low costs. In addition the fibers can be orientated to wind in a single direction or optionally in varying directions.

Alternatively, the implant formed of a fiber composite material can be prepared using a pultrusion method by saturating individual fibers or bundles of fibers with a resin, for example one the polymeric materials described above, and pulling the resin saturated fibers through a die to provide the profile of the desired implant. The resulting implant can be machined as described above to provide the final configuration including a threaded exterior, chamfer surfaces and openings. Implants prepared according the this pultrusion method generally have fibers orientated in the same direction, for example in an direction orientated to lie substantially parallel to the longitudinal axis, substantially perpendicular to the longitudinal axis or oblique to the longitudinal axis.

In yet another method the fiber reinforced composite can be prepared using chopped fibers, platelets, or particulates as reinforcing elements that have been embedded within a curable resin, for example one or more of the polymers described above. The reinforced material can be cured, molded and/or extruded according to techniques known in the art.

The osteogenic compositions used in this invention preferably comprise a therapeutically effective amount to stimulate or induce bone growth or healing of a substantially pure bone inductive factor such as a bone morphogenetic protein in a pharmaceutically acceptable carrier. The preferred osteoinductive factors include, but are not limited to, the recombinant human bone morphogenic proteins (rhBMPs) because they are available in unlimited supply and do not transmit infectious diseases. Most preferably, the bone morphogenetic protein is a rhBMP-2, rhBMP-4 or heterodimers thereof. The concentration of rhBMP-2 is generally between about 0.4 mg/ml to about 1.5 mg/ml, preferably near 1.5 mg/ml. However, any bone morphogenetic protein is contemplated including bone morphogenetic proteins designated as BMP-1 through BMP-13. BMPs are available from Genetics Institute, Inc., Cambridge, Massachusetts and may also be prepared by one skilled in the art as described in U.S. Patent Nos. 5,187,076 to Wozney et al.; 5,366,875 to Wozney et al.; 4,877,864 to Wang et al.: 5,108,922 to Wang et al.; 5,116,738 to Wang et al.; 5,013,649 to Wang et al.: 5,106,748 to Wozney et al.; and PCT Patent Nos. WO93/00432 to Wozney et al.: WO94/26893 to Celeste et al.; and WO94/26892 to Celeste et al. All osteoinductive factors are contemplated whether obtained as above or isolated from bone. Methods for isolating bone morphogenic protein from bone are described in U.S. Patent No. 4,294,753 to Urist and Urist et al., 81 PNAS 371, 1984.

The choice of carrier material for the osteogenic composition is based on the application desired, biocompatability, biodegradability, and interface properties. The bone growth inducing composition can be introduced into the cavity of the implant in any suitable manner. For example, the composition may be injected into the cavity or pressed into the cavity. Preferably the osteogenic composition is deposited into the cavity prior to implantation into the disc space. In other embodiments, the osteogenic composition injected or pressed into the cavity through one or more of the through-slits, or tool engagement openings that provide access to the cavity after the implant has been implantation in the disc space. The osteogenic factor, preferably a BMP, may be provided in freeze-dried form and reconstituted in a pharmaceutically acceptable liquid or gel carrier such as sterile water, physiological saline or any other suitable carrier. The carrier may be any suitable medium capable of delivering the proteins to the implant. Preferably the medium is supplemented with a buffer solution as is known in the art. In one specific embodiment of the invention, rhBMP-2 is suspended or admixed in a carrier, such as water, saline, liquid collagen or injectable bicalcium phosphate. In a most preferred embodiment, BMP is applied to the pores of the graft and then lypholized or freeze-dried. The graft-BMP composition can then be frozen for storage and transport. Alternatively, the osteoinductive protein can be added at the time of surgery.

Other osteoinductive protein carriers are available to deliver proteins to a chamber defined within the spacer or to locations around the implantation site of the bone material. Potential carriers include calcium sulphates, polylactic acids, polyanhydrides, collagen, calcium phosphates, polymeric acrylic esters and demineralized bone. The carrier may be any suitable carrier capable of delivering the proteins. Most preferably, the carrier is capable of being eventually resorbed into the body. One preferred carrier is an absorbable collagen sponge marketed by Integra LifeSciences Corporation under the trade name Helistat® Absorbable Collagen Hemostatic Agent. Another preferred carrier is an open cell polylactic acid polymer (OPLA). Other potential matrices for the compositions may be biodegradable and chemically defined calcium sulfates, calcium phosphates such as tricalcium phosphate (TCP) and hydroxyapatite (HA) and including injectable bicalcium phosphates (BCP), and polyanhydrides. Other potential materials are biodegradable and biologically derived, such as bone or dermal collagen. Further matrices are comprised of pure proteins or extracellular matrix components. The osteoinductive material may also be an admixture of BMP and a polymeric acrylic ester carrier, such as polymethylmethacrylic.

For packing the chambers of the implants of the present invention, the carriers are can be provided as a sponge, which can be compressed into the chamber, or as strips or sheets, which may be folded to conform to the chamber. Preferably, the carrier has a width and length, which are each slightly greater than the width and length of the chamber. In the most preferred embodiments, the carrier is soaked with a rhBMP-2 solution and then compressed into the chamber. The sponge is held within the chamber by the compressive forces provided by the sponge against the wall of the dowel. It may be preferable for the carrier to extend out of the openings of the chamber to facilitate contact of the osteogenic composition with the highly vascularized tissue surrounding the fusion site. The carrier can also be provided in several strips sized to fit within the chamber. The strips can be placed one against another to fill the interior. As with the folded sheet, the strips can be arranged within the implant in several orientations. Preferably, the osteogenic material, whether provided in a sponge, a single folded sheet or in several overlapping strips, has a length corresponding to the length and width of the chamber.

One carrier is a biphasic calcium phosphate ceramic. Hydroxyapatite/tricalcium phosphate ceramics are preferred because of their desirable bioactive properties and degradation rates in vivo. The preferred ratio of hydroxyapatite to tricalcium phosphate is between about 0:100 and about 65:35. Any size or shape ceramic carrier which will fit into the chambers defined in the load bearing member are contemplated. Ceramic blocks are commercially available from Sofamor Danek Group, B. P. 4-62180 Rang-du-Fliers, France and Bioland, 132 Route d:Espagne, 31100 Toulouse, France. Of course, rectangular and other suitable shapes are contemplated. The osteoinductive factor is introduced into the carrier in any suitable manner. For example, the carrier may be soaked in a solution containing the factor.

In the following, the invention will be described with the help of preferred embodiments referring to the drawings.

### BRIEF DESCRIPTION OF THE DRAWINGS

FIG. 1 is a perspective view of one embodiment of a vertebral implant according to the present invention.
FIG. 2 is an top plan view of the vertebral implant depicted to FIG. 1.
FIG. 3 is an elevated side view of the vertebral implant depicted in FIG. 1.
FIG. 4 is an elevated first end view of the vertebral implant depicted in FIG. 1.
FIG. 5 is an elevated second end view of the vertebral implant depicted in FIG. 1.
FIG. 6 is a top plan view of an alternative embodiment of a vertebral implant according to the present invention.
FIG. 7 is an elevated side view of the vertebral implant depicted in FIG. 6.
FIG. 8 is an elevated rear view of the vertebral implant depicted in FIG. 6.
FIG. 9 is an elevated front view of the vertebral implant depicted in FIG. 6
FIG. 10 is a cross-sectional view of a hollow vertebral implant according to the present invention.
FIG. 11 is a top view of a lumbar vertebra illustrating the bilateral placement of a pair of vertebral implants in according to the present invention.

### BRIEF DESCRIPTION OF THE PREFERRED EMBODIMENT

For the purposes of promoting an understanding of the principles of the invention, reference will now be made to the embodiments illustrated herein and specific language will be used to describe the same. It will nevertheless be understood that no limitation of the scope of the invention is thereby intended. Any alterations and further modifications in the described processes, systems or devices, and any further applications of the principles of the invention as described herein, are contemplated as would normally occur to one skilled in the art to which the invention relates.

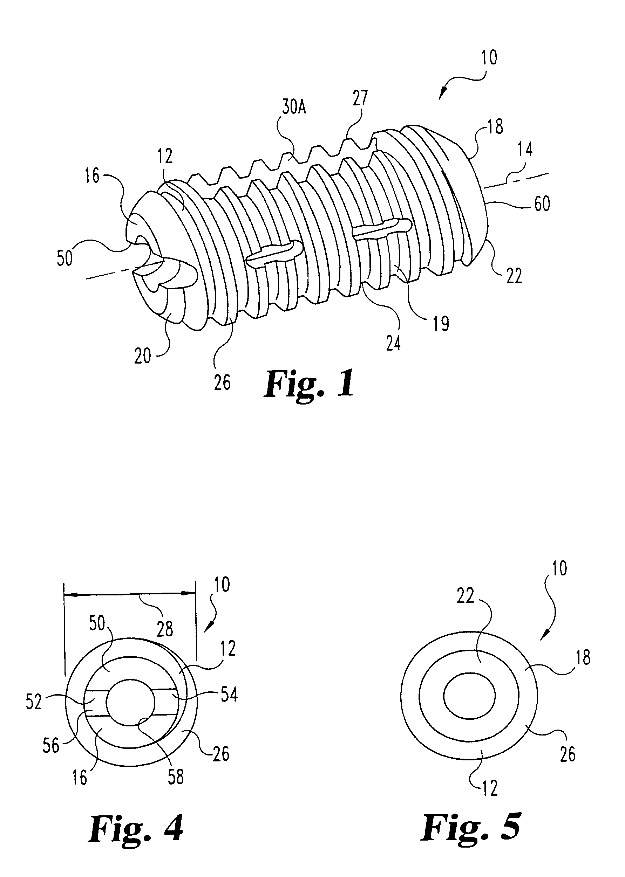

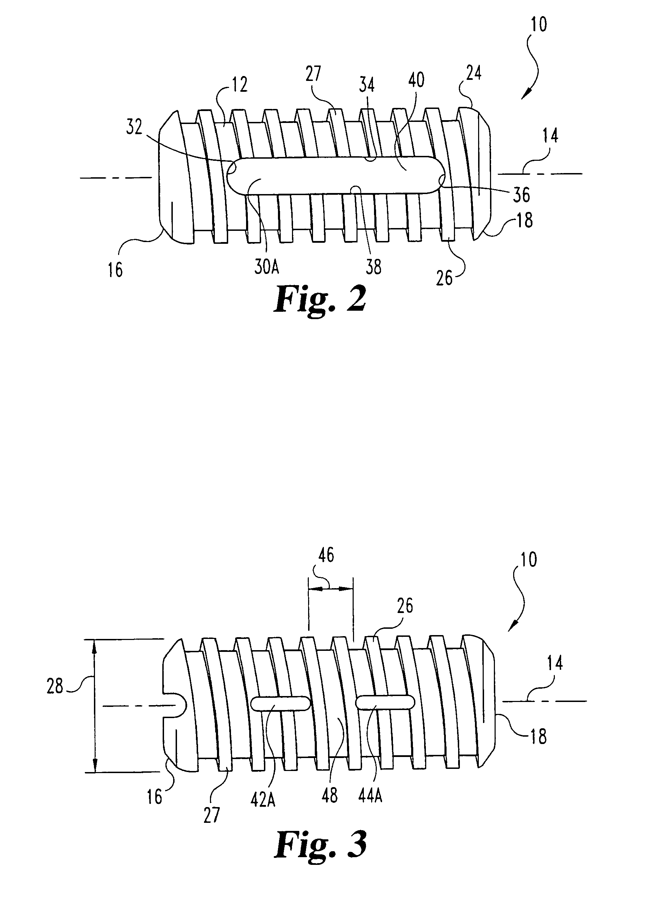

FIGS. 1 through 5 illustrate one embodiment of a vertebral implant 10 of the present invention. Implant 10 is a substantially solid implant having a substantially elongated cylindrical body 12. Implant 10 extends in an axial direction substantially symmetrically about a longitudinal axis 14 terminating at proximal end 16 and in the opposite axial direction at a distal end 18. In the illustrated embodiment, proximal end 16 is tapered by means of a first chamfer surface 20, which extends substantially about the periphery of proximal end 16. Similarly, a second chamfer surface 22 extends substantially about the periphery of distal end 18. Body 12 has a cylindrical wall 24 extending from first chamfer surface 20 to second chamfer surface 22 and defining an axial external thread 26.

In one form, external thread 26 extends from proximal end 16 of body 12 to a distal end 18. In alternative forms external thread 26 can extend over only a portion of body 12. Further, external thread 26 need not be positioned symmetrically about body 12. External thread 26 is provided to facilitate threading insertion of implant 10 into an intervertebral space between adjacent vertebra. To facilitate insertion of implant 10 into a intervertebral space, thread 26 is provided with a small lead angle; preferably less than or equal to about 10°. Thread 26 is configured to include a wide variety of shapes including either a sharp triangular or a rounded cross-sectional profile. However, it is preferable that thread 26 have a trapezoidal shaped cross-sectional profile. The thickness of thread 26 inhibits implant 10 from subsiding into the endplates of the vertebrae. The wide thread ribs 27 also reinforce the cylindrical wall 24. According to a preferred embodiment of the invention, the average cross-sectional thickness of thread 26 is between about 1/25 and about 1/15 of the overall length of implant 10; more preferable the cross-sectional thickness of thread 26 is about 1/20 of the overall length of implant 10.

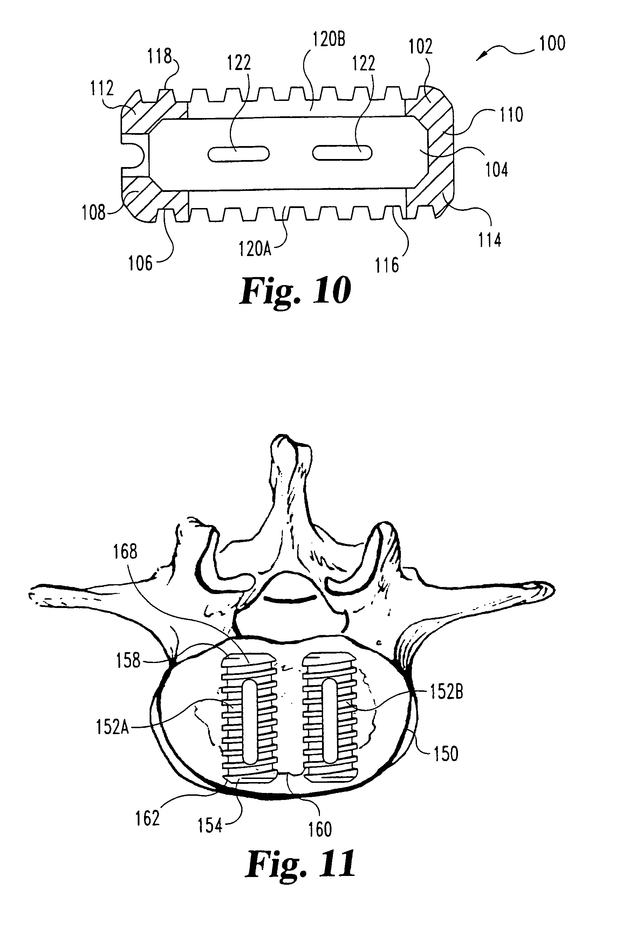

Implant 10 has a diameter, represented by reference line 28, selected to be suitable for implantation in the intervertebral space between adjacent vertebrae, including cervical, thoracic, lumbar and sacrum vertebrae. In preferred embodiments, implant 10 has an external diameter defined as the diameter of the external surface of thread 26, selected to be between about 10 mm and about 30 mm; more preferably between about 15 mm and about 25 mm. Implant 10 has a length measured along the longitudinal axis 14 of between about 20 mm and about 30 mm. Preferably implant 10 is selected to have a length so that a proximal end 16 bears against a portion of the cortical rim or the anterior peripheral apophyseal ring portion of adjacent vertebrae and while the distal end 18 bears against a portion of the posterior cortical rim or the apophyseal ring portion of the vertebrae. (See FIG. 11 discussed more fully below.)

A pair of elongated through-holes 30A are provided to extend through the external surface 19 of body 12. In the illustrated embodiment, through-holes 30A are positioned substantially diametrically opposed to each other about body 12. Through-holes 30A extend in the direction substantially parallel to longitudinal axis 14 and across several thread ribs 27 of thread 26, thus directly interrupting the course of thread 26. In the illustrated embodiment, through-holes 30A extend approximately over the entire length of base body 12. Thus the through-holes 30A extend across several thread ribs 27. The through-holes 30A are arranged symmetrically with respect to the longitudinal axis of body 12. The width of each of through-holes 30A can be variable. In preferred embodiments, the width of each of through-holes 30A are substantially the same and equal to approximately half of the diameter of the implant 10 as measured to include thread 26. Through-holes 30A extend through the body 12 and are aligned with each other to form a substantially smooth through bore--through body 12 so that a central cavity 40 is formed.

Cavity 40 is defined by internal walls 32, 34, 36, and 38, and approximates a parallelepiped extending transversely through body 12. The opposing axial walls 32 and 36 of cavity 3 are concave, while the opposing longitudinal walls 34 and 38 are substantially planar and parallel with each other. Cavity 40 is provided to allow a bone bridge to grow between adjacent vertebra and thus allows the adjacent vertebrae to fuse together.

Cylindrical wall 24 is also interrupted by two pairs of slits 42A and 44A. Slits 42A and 44A are axially displaced from each other along body 12. Each slit of the pair of slits 42A are positioned substantially diametrically opposed to each other about body 12. Similarly each slit of the second pair of slits 44A are positioned substantially diametrically opposed to each other about body 12. That is, each pair of slits, 42A and 44A are disposed symmetrically in body 12 about longitudinal axis 14. Each slit 42A and 44A are positioned about body 12 at an angular distance of about 90° from through-hole 30A. Each of slits 42A and 44A extend in the longitudinal direction, and each slit opens into cavity 40. Elongate slits 42A and 44A extend along the cylindrical wall 24 to interrupt thread 26. In one embodiment, slits, 42A and 44A are shorter and narrower than the through-holes 30A. The axial distance, illustrated by reference line 46, between pairs of slits 42A and 44A is selected provide a portion of the exterior surface 48 that includes at least two thread ribs 27 that are not interrupted by slits 42A and 44A. Preferably, slits 42A and 44A extend substantially the same length along longitudinal axis and each is substantially equal to about 1/3 to about 1/8 of the total longitudinal length of body 12.

Each of the slits 42A and 44A enable tissue lateral of implanted implant 10 to infuse into the interior of the implant 10.

Proximal end 16 terminates in a first chamfer surface 20, which extends substantially about the periphery of end 16. Chamfer surface 20 can be provided to bear against a portion of the apophyseal ring structure. Thus chamfer surface 20 can be provided to define a conical surface positioned at an angle oblique from longitudinal axis 14. In preferred embodiments chamfer surface 20 is selected to lie at an angle between about 5° and about 60° from the longitudinal axis.

End cap 50 closes proximal end 16. End cap 50 is includes at least one tool engaging portion 52 for mating engaging with a manipulation tool (not shown). In preferred embodiments, the tool engaging structure 52 comprises grooves 54 and 56 extending a radial direction and positioned diametrically opposed to each other about longitudinal axis 14. Corresponding convex counterparts of a manipulation tool can matingly engage with grooves 54 and 56 so that rotation of the tool transfers the torque required in the longitudinal direction of implant 10 to screw (or thread) implant 10 into the intervertebral space. In addition manipulation tool can be used to orientate implant 10 within the intervertebral space to align through-holes 30A to contact and oppose the upper and lower vertebral end plates.

End cap 50 can also include at least one threaded bore 58 having internal threads that engage in a corresponding threaded projection on a manipulation tool (not shown). Threaded bore 58 can, but is not required to, extend through end cap 50 and provides additional access to the interior cavity 40 of body 12 to allow for the infusion of bony tissue and facilitate fusion of the adjacent vertebrae.

Distal end 18 is tapered by means of a second chamfer surface 22, which extends substantially about the periphery of distal end 18. The chamfer surface 22 on distal end 18 extends in an axial direction tapering axially from the outer diameter of the distal end 18 and defines an insertion end 60. Insertion end 60 can be provided in a variety of configurations, for example conical, rounded, curved, frustoconicol and the like. The length of insertion end 60 is preferably at least 1/10 of the length of body 12 measured along the length of body 12 that is substantially cylindrical. In one form, insertion end 60 is not provided with an external thread to facilitate insertion of implant 10 into a vertebral space. Thus the implant 10 can be easily driven into the intervertebral space to spread the vertebrae, if necessary. Once the implant is embedded to a depth that thread 26 contacts the vertebra, implant 10 can be rotated to engage thread 26 with the vertebra. Then implant 10 can be positioned accurately in the intervertebral space by screwing it in the axial direction, without a substantial risk that implant 10 will penetrate the spinal cord other tissues and organs. In alternative forms, insertion end 60 can be provided with an external thread.

In.the illustrated embodiment, distal end 18 and insertion end 60 are a one piece integral portion of body 12. In alternative embodiments, insertion end 60 is separable from distal end 18. For example, insertion end 60 can include an external threaded portion and be threadedly engaged to corresponding internal threads on distal end 18. Alternatively, insertion end 10 can be press fitted on distal end 18.

End cap 50 and/or insertion end 60 are preferably formed to provide a thick bearing or support structure for implant 10. End cap 50 and insertion end 60 are provided to be between about 1.5 times to about 4 times as thick as the cylindrical wall 24. The thickened ends provide benefits for bone fusion. The thicker ends maintain desired disk space height and orientation while the less thick cylindrical wall 24 does not stress shield the endplates of the adjacent vertebrae. Less stress shielding accelerates bone ingrowth into implant 10 and the resulting new bone exhibits stronger, load supporting tissue associated with cortical bone. The results are particularly pronounced when implant 10 is formed a synthetic material as described more fully below.

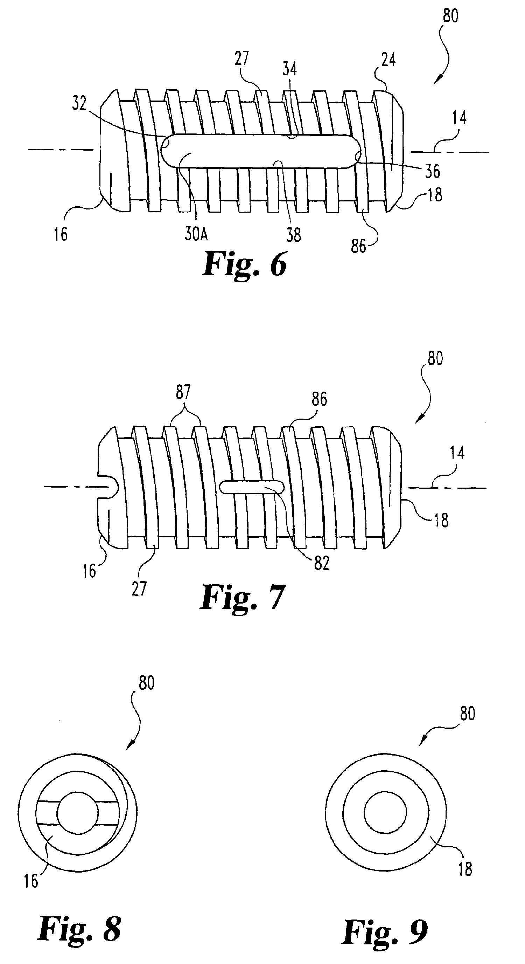

FIGS. 6 to 9 illustrate an alternative embodiment of a threaded vertebral implant 80 for use with this invention. The features corresponding to the implant 10 depicted in FIGS. 1-5 are denoted by like reference numbers. In this embodiment, the external surface of implant is interrupted by a through-holes 30A and a pair diametrically opposed slits 82 extending in the axial of longitudinal direction. Each of slits 82 also interrupt external thread 86. Each slit 82 extend continuously across at least two ribs 87 of thread 86.

FIG. 10 illustrates a cross sectional view of a hollow implant 100 for this invention. Implant 100 can be provided with a substantially cylindrical body 102 having a hollow interior or cavity 104. The hollow cavity 104 extends in the longitudinal direction and is defined by an internal, substantially cylindrical wall 106 and terminates in an axial direction at a proximal end 108 and at an opposite distal end 110. End cap 112 closes proximate end 108; however, when desired end cap 112 can be separated from proximate end 108 to allow access to cavity 104. Similarly, insertion end cap 114 closes distal end 110. Separation of one or both end cap 112 and insertion end cap 114 from body 102 allows access to cavity 104 to facilitate loading of an osteogenic composition into implant 100.

Hollow implant 100 is provided substantially as described for implants 1 and 80 described above. Since implant 100 is hollow it provides significantly more internal area to serve as a depot for biological material such as an osteogenic composition, antibiotics or other desired pharmaceutical preparations.

Implant 100 has an exterior surface 116 provided substantially as described for implant 10 and 80 including threads 118, through-holes 120A and 120B and slits 122. Through-holes 120A and 120B provide an opening into cavity 104. In addition, first end cap 112 and insertion end 114 can include one or more openings into cavity 104.

FIG. 11 is a top plan view looking down on the superior endplate of a lumbar vertebra 150 and of a pair of implants 152A and 152B provided according to this invention, and illustrates the bi-laterally placement of a pair of implants. Implant 152A and 152B have been inserted from a posterior approach. However, implants 152A and 152B can be inserted either from a lateral posterior direction or an anterior direction or a lateral anterior direction. Further while implants 152 A/B are illustrated to lie substantially parallel with each other, it will be understood by those skilled in the art that the implants can be placed at an oblique angle relative to each other as referenced by their respective longitudinal axis. Implants 152A/B are substantially identical in configuration and size. Although for optimal treatment of specific deformities and/or diseases, the implants need not be identical either in configuration or size.

Referring specifically to implant 152A, in a preferred embodiment, this elongate implant is sized so that proximate end 154 bears against a portion of the apophyseal ring portion 160 while the distal end 158 bears against a portion of the posterior apophyseal ring 160 of the vertebra 150 on an opposite side of the vertebral endplate. This effectively resist the tendency implant 152A from receding into the less hard spongy bone tissue that can be exposed upon preparing a suitable cavity in the disk space for the implantation of the implants of this invention.

The chamfered surfaces 162 and 168 of proximal end 154 and distal end 158, respectively, provide surfaces that matingly bear against a portion of the apophyseal ring structure. Thus, the angle of chamfer can be selected to be enhance engagement with the periphery ring structure of the endplates. This periphery of the vertebrae can provide a thicker cortical bone surface that can withstand greater loading. By lodging the chamfer surfaces 162 and 168 against this cortical bone tissue, implant 152A is effectively secured in position in the intervertebral space. Further the chamfer surfaces 162, 164 in conjunction with the thickened ends 154 and 158 provide sufficient biomechanical strength for the implant to withstand the loads exerted on the spinal column through a normal or suggested course of patent physical activity without damaging either the implant or the adjacent vertebrae. This beneficial result is particularly pronounced when the implant of formed of a synthetic organic material as discussed more fully above.

The present invention contemplates modifications as would occur to those skilled in the art. It is also contemplated that processes embodied in the present invention can be altered, rearranged, substituted, deleted, duplicated, combined, or added to other processes as would occur to those skilled in the art without departing from the present invention. In addition, the various stages, steps, procedures, techniques, phases, and operations within these processes may be altered, rearranged, substituted, deleted, duplicated, or combined as would occur to those skilled in the art.

## Claims

1. A vertebral implant (10,80,100,152A,152B) for implantation into an intervertebral space said implant comprising:
an elongate body (12,102) having a proximal end (16,108,154) and a distal end (18,110,158) and outer substantially cylindrical wall extending therebetween, said wall defining external screw threads (26,86,118);
said proximal end (16,108,154), comprising a first chamfer surface (20,162) and an end cap (50,112) having tool engaging structures (52) formed therein;
said distal end (18,110,158) defining a second chamfer surface (22,168), and wherein said implant (10,80,100,152A,152B) is formed of a synthetic, non-metallic material, **characterized in that** the first and second chamfer surfaces (20,162,22,168) extend substantially about the periphery of proximal end (16,108,154) and distal end (18,110,158), respectively.

2. The implant (10,80,100,152A,152B) of claim 1 wherein said body (12,102) is sized to extend substantially across a vertebral endplate of the vertebra (150) so said first chamfer surface (20,162) bears against a first portion of a apophyseal ring of the vertebral endplate while said second chamfer surface (22,168) bears against a second opposite portion of the apophyseal ring of adjacent vertebrae (150).

3. The implant (10,80,100,152A,152B) of claim 1 or 2 wherein the wall includes a first opening (30A,120A) formed therethrough and an opposite second opening (30B,120B) formed therethrough.

4. The implant (10,80,100,152A,152B) of claim 1 wherein end cap (50,112) defines the first chamfer surface (20,162).

5. The implant (10,80,100,152A,152B) of claim 1 wherein the end cap (50,112) is separable from the elongate body (12,102).

6. The implant (10,80,100,152A,152B) of claim 1 wherein the second chamfer surface (22,162) defines a rounded, frustoconical, conical, tapered, or convex surface portion.

7. The implant (10,80,100,152A,152B) of claim 1 wherein the end cap (50,112) is thicker than said cylindrical wall.

8. The implant (10,80,100,152A,152B) of claim 1 wherein the synthetic, non-metallic material is a biodegradable material.

9. The implant (10,80,100,152A,152B) of claim 1 wherein the synthetic, non-metallic material is a composite material.

10. The implant (10,80,100,152A,152B) of claim 1 wherein the synthetic, non-metallic material is a ceramic material.

11. The implant (10,80,100,152A,152B) of claim 1 comprising at least two elongated holes (30A,120A,120B) located on said wall diametrically opposite each other and extending over a plurality of screw ribs.

12. The implant (10,80,100,152A,152B) of claim 11 comprising at least two longitudinal slits (42A,82,122) located in said wall about 90° from said elongated holes, each of the at least two longitudinal slits (42A,82,122) interrupting at least one thread rib.

13. The implant (10,80,100,152A,152B) of claim 11 comprising two longitudinal slits (42A,44B,122) axially spaced from each other on said wall to allow at least one uninterrupted thread rib therebetween.

14. The implant (10,80,100,152A,152B) of claim 1 wherein said external thread (26,86,118) defines a thread rib having a trapezoidal cross-section.

15. The implant (10,80,100,152A,152B) of claim 1 wherein the elongate body (12,102) comprises a tool-engaging portion (52).

16. The implant (10,80,100,152A,152B) of claim 15 wherein the tool-engaging portion comprises (52) two grooves (54,56) formed in said proximal end (16,108,154) and extending in a radial direction.

17. The implant (10,80,100,152A,152B) of claim 15 wherein the tool-engaging portion (52) comprises a threaded bore (58).

18. The implant (10,80,100,152A,152B) of claim 1 wherein the non-metallic material is selected from the group of material consisting of: polyanhydrides; polyamides, poly(amino acids), polycaprolactones, polylactate, poly(lactide-co-glycolide); polyorthoesters; acrylics; polycarbonates; polyesters; polyethers, poly(ether ketone); poly(ether, ether ketone); poly(aryl ether ketones); poly(ether ether ketone ether ketone); poly(ethylene terephthalate), poly(methyl (meth)acrylate), polyolefins, polysulfones, polyurethane; poly(vinyl chloride), carbon fiber reinforced composite, glass fiber reinforced composite, and mixtures thereof.

19. The implant (10,80,100,152A,152B) of claim 1 wherein the non-metallic material comprises elongated carbon fibers.

20. The implant (10,80,100,152A,152B) of claim 1 wherein the non-metallic material is selected from the group consisting of: hydroxylapatite; alumina, zirconia and mixtures thereof.

21. A method of preparing a implant (10,80,100,152A,152B), said method comprising:
selecting synthetic a non-metallic material;
forming an elongate body (12,102) having a proximal end (16,108,154) and a distal end (18,110,158) and outer substantially cylindrical wall extending therebetween from said synthetic, non-metallic material;
providing an external thread (26,86,118) on said elongate body (12,102);
chamfering a portion of said proximal end (16,108,154) and said distal end (18,110,158), thereby providing first and second chamfer surfaces (20,162,22,168)
and providing an end cap (50, 112) on the proximal end (16,108,154), the end cap (50,112) being provided with tool engaging structures (52) formed therein, **characterized in that** the first and second chamfer surfaces (20,162,22, 168) extend substantially about the periphery of proximal end (16,108,154) and distal end (18,110,158), respectively.

22. The method of claim 21 wherein the non-metallic material is selected from the group consisting of: polyanhydrides; polyamides, poly(amino acids), polycaprolactones, polylactate, poly(lactide-co-glycolide); polyorthoesters; acrylics; polycarbonates; polyesters; polyethers, poly(ether ketone); poly(ether, ether ketone); poly(aryl ether ketones); poly(ether ether ketone ether ketone); poly(ethylene terephthalate), poly(methyl (meth)acrylate), polyolefins, polysulfones, polyurethane; poly(vinyl chloride), carbon fiber reinforced composite, glass fiber reinforced composite, and mixtures thereof.

23. The method of claim 21 wherein the synthetic, non-metallic material is selected from the group consisting of: hydroxylapatite; alumina, zirconia and mixtures thereof.

24. The method of claim 21 wherein said forming comprises winding a resin impregnated fiber about a spindle.

25. The method of claim 21 wherein said forming includes pultrusion fabrication techniques.

26. The method of claim 21 wherein said forming comprises extruding a polymeric material.

## Patentansprüche

1. Wirbelimplantat (10, 80, 100, 152A, 152B) zum Implantieren in einen Bandscheibenraum, wobei das Implantat aufweist:
einen länglichen Körper (12, 102), der ein proximales Ende (16, 108, 154) und ein distales Ende (18, 110, 158) und eine äußere, im Wesentlichen zylinderförmige Wand aufweist, die sich dazwischen erstreckt, wobei die Wand Schrauben-Außengewinde (26, 86, 118) definiert,
wobei das proximale Ende (16, 108, 154) eine erste Fasen-Fläche (20, 162) und eine Endkappe (50, 112) mit darin ausgebildeten Werkzeug-Eingriffs-Strukturen (52) aufweist,
wobei das distale Ende (18, 110, 158) eine zweite Fasen-Fläche (22, 168) definiert, und wobei das Implantat (10, 80, 100, 152A, 152B) aus einem synthetischen, nicht-metallischen Material gebildet ist, **dadurch gekennzeichnet, dass** sich die erste und die zweite Fasen-Fläche (20, 162, 22, 168) im Wesentlichen um den Umfang des proximalen Endes (16, 108, 154) beziehungsweise des distalen Endes (18, 110, 158) erstrecken.

2. Implantat (10, 80, 100, 152A, 152B) gemäß Anspruch 1, wobei der Körper (12, 102) derart bemessen ist, dass er sich im Wesentlichen über eine Wirbel-Endplatte des Wirbels (150) erstreckt, so dass die erste Fasen-Fläche (20, 162) gegen einen ersten Abschnitt eines Apophysenrings der Wirbel-Endplatte drückt, während die zweite Fasen-Fläche (22, 168) gegen einen zweiten entgegengesetzten Abschnitt des Apophysenrings eines benachbarten Wirbels (150) drückt.

3. Implantat (10, 80, 100, 152A, 152B) gemäß Anspruch 1 oder 2, wobei die Wand eine erste Öffnung (30A, 120A), die durch diese hindurch ausgebildet ist, und eine gegenüberliegende zweite Öffnung (30A, 120B), die durch diese hindurch ausgebildet ist, aufweist.

4. Implantat (10, 80, 100, 152A, 152B) gemäß Anspruch 1, wobei die Endkappe (50, 112) die erste Fasen-Fläche (20, 162) definiert.

5. Implantat (10, 80, 100, 152A, 152B) gemäß Anspruch 1, wobei die Endkappe (50, 112) von dem länglichen Körper (12, 102) abtrennbar ist.

6. Implantat (10, 80, 100, 152A, 152B) gemäß Anspruch 1, wobei die zweite Fasen-Fläche (22, 162) einen runden, kegelstumpfartigen, konischen, verjüngten oder konvexen Oberflächenabschnitt definiert.

7. Implantat (10, 80, 100, 152A, 152B) gemäß Anspruch 1, wobei die Endkappe (50, 112) dicker als die zylinderförmige Wand ist.

8. Implantat (10, 80, 100, 152A, 152B) gemäß Anspruch 1, wobei das synthetische nicht-metallische Material ein biologisch abbaubares Material ist.

9. Implantat (10, 80, 100, 152A, 152B) gemäß Anspruch 1, wobei das synthetische nicht-metallische Material ein Verbundwerkstoff ist.

10. Implantat (10, 80, 100, 152A, 152B) gemäß Anspruch 1, wobei das synthetische nicht-metallische Material ein Keramikmaterial ist.

11. Implantat (10, 80, 100, 152A, 152B) gemäß Anspruch 1, das mindestens zwei längliche Löcher (30A, 120A, 120B) aufweist, die einander diametral gegenüber an der Wand positioniert sind und sich über eine Mehrzahl von Schraubenrippen erstrecken.

12. Implantat (10, 80, 100, 152A, 152B) gemäß Anspruch 11, das mindestens zwei Längsschlitze (42A, 82, 122) aufweist, die um 90° von den länglichen Löchern in der Wand angeordnet sind, wobei jeder der mindestens zwei Längsschlitze (42A, 82, 122) mindestens eine Gewinderippe unterbricht.

13. Implantat (10, 80, 100, 152A, 152B) gemäß Anspruch 11, das zwei Längsschlitze (42A, 44B, 122) aufweist, die an der Wand axial im Abstand voneinander angeordnet sind, um mindestens eine nicht unterbrochene Gewinderippe dazwischen zu ermöglichen.

14. Implantat (10, 80, 100, 152A, 152B) gemäß Anspruch 1, wobei das Außengewinde (26, 86, 118) eine Gewinderippe mit einem trapezförmigen Querschnitt definiert.

15. Implantat (10, 80, 100, 152A, 152B) gemäß Anspruch 1, wobei der längliche Körper (12, 102) einen Werkzeug-Eingriffs-Abschnitt (52) aufweist.

16. Implantat (10, 80, 100, 152A, 152B) gemäß Anspruch 15, wobei der Werkzeug-Eingriffs-Abschnitt (52) zwei Nuten (54, 56) aufweist, die in dem proximalen Ende (16, 108, 154) ausgebildet sind und sich in einer radialen Richtung erstrecken.

17. Implantat (10, 80, 100, 152A, 152B) gemäß Anspruch 15, wobei der Werkzeug-Eingriffs-Abschnitt (52) eine Gewindebohrung (58) aufweist.

18. Implantat (10, 80, 100, 152A, 152B) gemäß Anspruch 1, wobei das nicht-metallische Material ausgewählt ist aus der Materialgruppe bestehend aus Polyanhydriden, Polyamiden, Poly(Aminosäuren), Polycaprolactonen, Polylactaten, Poly(lactid-co-glycolid), Polyorthoestern, Acrylen, Polycarbonaten, Polyestern, Polyethern, Poly(etherketon), Poly(ether, etherketon), Poly(aryletherketonen), Poly(ether etherketon etherketon); Poly(ethylenterephthalat), Poly(methyl (meth)acrylat), Polyolefinen, Polysulfonen, Polyurethan, Poly(vinylchlorid), einem kohlenstofffaserverstärkten Gemisch, einem glasfaserverstärkten Gemisch und Zusammensetzungen davon.

19. Implantat (10, 80, 100, 152A, 152B) gemäß Anspruch 1, wobei das nicht-metallische Material längliche Kohlenstoff-Fasern aufweist.

20. Implantat (10, 80, 100, 152A, 152B) gemäß Anspruch 1, wobei das nicht-metallische Material aus der Gruppe bestehend aus Hydroxylapatit, Aluminiumoxid, Zirkonoxid und Zusammensetzungen davon ausgewählt ist.

21. Verfahren zum Anfertigen eines Implantats (10, 80, 100, 152A, 152B), wobei das Verfahren aufweist:
Auswählen eines synthetischen, nicht-metallischen Materials,
Bilden eines länglichen Körpers (12, 102) aus dem synthetischen nicht-metallischen Material, der ein proximales Ende (16, 108, 154) und ein distales Ende (18, 110, 158) und eine äußere, im Wesentlichen zylinderförmige Wand aufweist, die sich dazwischen erstreckt,
Bereitstellen eines Außengewindes (26, 86, 118) an dem länglichen Körper (12, 102),
Abfasen eines Abschnitts des proximalen Endes (16, 108, 154) und des distalen Endes (18, 110, 158), wodurch eine erste und eine zweite Fasen-Fläche (20, 162, 22, 168) bereitgestellt werden, und Bereitstellen einer Endkappe (50, 112) an dem proximalen Ende (16, 108, 154), wobei die Endkappe (50, 112) mit darin ausgebildeten Werkzeug-Eingriffs-Strukturen (52) vorgesehen ist, **dadurch gekennzeichnet, dass** sich die erste und die zweite Fasen-Fläche (20, 162, 22, 168) im Wesentlichen um den Umfang des proximalen Endes (16, 108, 154) beziehungsweise des distalen Endes (18, 110, 158) erstrecken.

22. Verfahren gemäß Anspruch 21, wobei das nicht-metallische Material aus der Gruppe bestehend aus Polyanhydriden, Polyamiden, Poly(Aminosäuren), Polycaprolactonen, Polylactaten, Poly(lactid-co-glycolid), Polyorthoestern, Acrylen, Polycarbonaten, Polyestern, Polyethern, Poly(etherketon), Poly(ether, etherketon), Poly(aryletherketonen), Poly(ether etherketon etherketon), Poly(ethylenterephthalat), Poly(methyl (meth)acrylat), Polyolefinen, Polysulfonen, Polyurethan, Poly(vinylchlorid), einem kohlenstofffaserverstärkten Gemisch, einem glasfaserverstärkten Gemisch und Zusammensetzungen davon ausgewählt wird.

23. Verfahren gemäß Anspruch 21, wobei das synthetische nicht-metallische Material aus der Gruppe bestehend aus Hydroxylapatit, Aluminiumoxid, Zirkonoxid und Zusammensetzungen davon ausgewählt wird.

24. Verfahren gemäß Anspruch 21, wobei das Ausbilden das Wickeln einer harzgetränkten Faser um eine Spindel aufweist.

25. Verfahren gemäß Anspruch 21, wobei das Ausbilden Pultrusions-Herstellungstechniken miteinschließt.

26. Verfahren gemäß Anspruch 21, wobei das Ausbilden das Extrudieren eines Polymer-Materials aufweist.

## Revendications

1. Implant vertébral (10, 80, 100, 152A, 152B), pour l'implantation dans un espace intervertébral, ledit implant comprenant :
un corps allongé (12, 102) ayant une extrémité proximale (16, 108, 154) et une extrémité distale (18, 110, 158) et une paroi externe sensiblement cylindrique s'étendant entre elles, ladite paroi définissant des filetages de vis externes (26, 86, 118) ;
ladite extrémité proximale (16, 108, 154) comprenant une première surface de chanfrein (20, 162) et un capuchon d'extrémité (50, 112) ayant des structures de mise en prise d'outil (52) formées dans celui-ci ;
ladite extrémité distale (18, 110, 158) définissant une seconde surface de chanfrein (22, 168), et dans lequel ledit implant (10, 80, 100, 152A, 152B) est formé avec un matériau synthétique non métallique, **caractérisé en ce que** les première et seconde surfaces de chanfrein (20, 162, 22, 168) s'étendent sensiblement autour de la périphérie de l'extrémité proximale (16, 108, 154) et de l'extrémité distale (18, 110, 158), respectivement.

2. Implant (10, 80, 100, 152A, 152B) selon la revendication 1, dans lequel ledit corps (12, 102) est dimensionné pour s'étendre sensiblement sur une plaque d'extrémité vertébrale de la vertèbre (150) de sorte que ladite première surface de chanfrein (20, 162) s'appuie contre une première partie d'un anneau apophysaire de la plaque d'extrémité vertébrale alors que la seconde surface de chanfrein (22, 168) s'appuie contre une seconde partie opposée de l'anneau apophysaire des vertèbres (150) adjacentes.

3. Implant (10, 80, 100, 152A, 152B) selon la revendication 1 ou 2, dans lequel la paroi comprend une première ouverture (30A, 120A) formée à travers celle-ci et une seconde ouverture (30B, 120B) opposée formée à travers celle-ci.

4. Implant (10, 80, 100, 152A, 152B) selon la revendication 1, dans lequel le capuchon d'extrémité (50, 112) définit la première surface de chanfrein (20, 162).

5. Implant (10, 80, 100, 152A, 152B) selon la revendication 1, dans lequel le capuchon d'extrémité (50, 112) peut être séparé du corps allongé (12, 102).

6. Implant (10, 80, 100, 152A, 152B) selon la revendication 1, dans lequel la seconde surface de chanfrein (22, 162) définit une partie de surface arrondie, tronconique, conique, progressivement rétrécie ou convexe.

7. Implant (10, 80, 100, 152A, 152B) selon la revendication 1, dans lequel le capuchon d'extrémité (50, 112) est plus épais que ladite paroi cylindrique.

8. Implant (10, 80, 100, 152A, 152B) selon la revendication 1, dans lequel le matériau non métallique synthétique est un matériau biodégradable.

9. Implant (10, 80, 100, 152A, 152B) selon la revendication 1, dans lequel le matériau synthétique non métallique est un matériau composite.

10. Implant (10, 80, 100, 152A, 152B) selon la revendication 1, dans lequel le matériau synthétique non métallique est un matériau en céramique.

11. Implant (10, 80, 100, 152A, 152B) selon la revendication 1, comprenant au moins deux trous allongés (30A, 120A, 120B) situés sur ladite paroi, diamétralement opposés l'un par rapport à l'autre et s'étendant sur une pluralité de nervures de vis.

12. Implant (10, 80, 100, 152A, 152B) selon la revendication 11, comprenant au moins deux fentes longitudinales (42A, 82, 122) situées dans ladite paroi environ à 90° desdits trous allongés, chacune des au moins deux fentes longitudinales (42A, 82, 112) interrompant au moins une nervure de filetage.

13. Implant (10, 80, 100, 152A, 152B) selon la revendication 11, comprenant deux fentes longitudinales (42A, 44B, 122) espacées axialement l'une de l'autre sur ladite paroi pour permettre au moins une nervure de filetage non interrompue entre elles.

14. Implant (10, 80, 100, 152A, 152B) selon la revendication 1, dans lequel ledit filetage externe (26, 86, 118) définit une nervure de filetage ayant une section transversale trapézoïdale.

15. Implant (10, 80, 100, 152A, 152B) selon la revendication 1, dans lequel le corps allongé (12, 102) comprend une partie de mise en prise d'outil (52).

16. Implant (10, 80, 100, 152A, 152B) selon la revendication 15, dans lequel la partie de mise en prise d'outil (52) comprend deux rainures (54, 56) formées dans ladite extrémité proximale (16, 108, 154) et s'étendant dans une direction radiale.

17. Implant (10, 80, 100, 152A, 152B) selon la revendication 15, dans lequel la partie de mise en prise d'outil (52) comprend un alésage fileté (58).

18. Implant (10, 80, 100, 152A, 152B) selon la revendication 1, dans lequel le matériau non métallique est choisi dans le groupe de matériaux comprenant : les polyanhydrides ; les polyamides, les acides polyaminés, les polycaprolactones, le polylactate, le polylactide-co-glycolide ; polyorthoesters ; les acryliques ; les polycarbonates ; les polyesters, les polyéthers, les polyéthercétones ; les polyétheréthercétones, les polyaryléthercétones ; les polyétheréthercétoneéthercétones ; les polyéthylènetéréphtalates, les polyméthylméthacrylates, les polyoléfines, les polysulfones, le polyuréthane ; le polychlorure de vinyle, les composites renforcés à la fibre de carbone, les composites renforcés à la fibre de verre, et leurs mélanges.

19. Implant (10, 80, 100, 152A, 152B) selon la revendication 1, dans lequel le matériau non métallique comprend des fibres de carbone allongées.

20. Implant (10, 80, 100, 152A, 152B) selon la revendication 1, dans lequel le matériau non métallique est choisi dans le groupe comprenant : l'hydroxylapatite ; l'alumine, la zircone et leurs mélanges.

21. Procédé pour préparer un implant (10, 80, 100, 152A, 152B), ledit procédé comprenant les étapes consistant à :
sélectionner un matériau synthétique non métallique ;
former un corps allongé (12, 102) ayant une extrémité proximale (16, 108, 154) et une extrémité distale (18, 110, 158) et une paroi externe sensiblement cylindrique s'étendant entre elles, à partir dudit matériau synthétique non métallique ;
prévoir un filetage externe (26, 86, 118) sur ledit corps allongé (12, 102) ;
chanfreiner une partie de ladite extrémité proximale (16, 108, 154) et de ladite extrémité distale (18, 110, 158), fournissant ainsi des première et seconde surfaces de chanfrein (20, 162, 22, 168) et fournissant un capuchon d'extrémité (50, 112) sur l'extrémité proximale (16, 108, 154), le capuchon d'extrémité (50, 112) étant prévu avec des structures de mise en prise d'outil (52) formées à l'intérieur de celui-ci, **caractérisé en ce que** les première et seconde surfaces de chanfrein (20, 162, 22, 168) s'étendent sensiblement autour de la périphérie de l'extrémité proximale (16, 108, 154) et de l'extrémité distale (18, 110, 158), respectivement.

22. Procédé selon la revendication 21, dans lequel le matériau non métallique est choisi dans le groupe comprenant : les polyanhydrides ; les polyamides, les acides polyaminés, les polycaprolactones, le polylactate, le polylactide-co-glycolide ; les polyorthoesters ; les acryliques ; les polycarbonates ; les polyesters, les polyéthers, les polyéthercétones ; les polyétheréthercétones, les polyaryléthercétones ; les polyétheréthercétoneéthercétones ; les polyéthylènetéréphtalates, les polyméthylméthacrylates, les polyoléfines, les polysulfones, le polyuréthane ; le polychlorure de vinyle, les composites renforcés à la fibre de carbone, les composites renforcés à la fibre de verre, et leurs mélanges.

23. Procédé selon la revendication 21 dans lequel le matériau synthétique non métallique est choisi dans le groupe comprenant : l'hydroxylapatite ; l'alumine, la zircone et leurs mélanges.

24. Procédé selon la revendication 21, dans lequel ladite étape de formation comprend l'étape consistant à enrouler une résine imprégnée de fibres autour d'une broche.

25. Procédé selon la revendication 21, dans lequel ladite étape de formation comprend des techniques de fabrication d'extrusion par étirage.

26. Procédé selon la revendication 21, dans lequel ladite étape de formation comprend l'étape consistant à extruder un matériau polymère.
